# EUROPÄISCHE PATENTSCHRIFT

(11) **EP 3 105 316 B1**
(45) Veröffentlichungstag und Bekanntmachung des Hinweises auf die Patenterteilung: **02.01.2019**
(21) Anmeldenummer: 15706193.8
(22) Anmeldetag: 16.02.2015
(51) Int. Cl.: C12N 7/00, C12N 5/077

(54) **IMMORTALISIERTE ZELLE**
IMMORTALIZED CELL
CELLULE IMMORTALISÉE

(30) Priorität: 14.02.2014 DE 102014101862
(43) Veröffentlichungstag der Anmeldung: 21.12.2016
(73) Patentinhaber: Eberhard Karls Universität Tübingen Medizinische Fakultät, 72074 Tübingen (DE)
(72) Erfinder: ALEXANDER-FRIEDRICH, Dorothea, 70565 Stuttgart (DE)
(74) Vertreter: Witte, Weller & Partner Patentanwälte mbB
(86) Internationale Anmeldenummer: PCT/EP2015/053213
(87) Internationale Veröffentlichungsnummer: WO 2015/121470

(56) Entgegenhaltungen:
- EP-A1- 1 207 196
- WO-A1-00/21523
- WO-A1-99/39724
- DARIMONT CHRISTIAN ET AL: "SV40 T antigen and telomerase are required to obtain immortalized human adult bone cells without loss of the differentiated phenotype", CELL GROWTH AND DIFFERENTIATION, Bd. 13, Nr. 2, Februar 2002 (2002-02), Seiten 59-67, XP002737735, ISSN: 1044-9523
- XIAOXUE Y ET AL: "Immortalization of human osteoblasts by transferring human telomerase reverse transcriptase gene", BIOCHEMICAL AND BIOPHYSICAL RESEARCH COMMUNICATIONS, ACADEMIC PRESS INC. ORLANDO, FL, US, Bd. 315, Nr. 3, 12. März 2004 (2004-03-12) , Seiten 643-651, XP004490112, ISSN: 0006-291X, DOI: 10.1016/J.BBRC.2004.01.102
- ENYI HUANG ET AL: "Conditionally Immortalized Mouse Embryonic Fibroblasts Retain Proliferative Activity without Compromising Multipotent Differentiation Potential", PLOS ONE, Bd. 7, Nr. 2, 23. Februar 2012 (2012-02-23), Seite e32428, XP055178777, DOI: 10.1371/journal.pone.0032428

## Beschreibung

Die vorliegende Erfindung betrifft eine immortalisierte Schädelperiostzelle, eine diese immortalisierte Schädelperiostzelle aufweisende immortalisierte Zelllinie, eine Zellkultur, die die immortalisierte Zelle oder Zelllinie aufweist, sowie ein Verfahren zur Herstellung einer immortalisierten Schädelperiostzelle.

In der Grundlagenforschung werden für *in vitro* Experimente größere Mengen von Zellmaterial benötigt, um beispielsweise funktionelle Assays durchzuführen oder das Adhäsions- und Proliferationsverhalten, die Gen- und Proteinexpression zu analysieren. Auch in anwendungsorientierten Disziplinen der Lebendwissenschaften besteht der Bedarf an Zellmaterial, beispielsweise für das sogenannte Tissue-Engineering oder die Zelltherapie.

Da Zellspender nicht permanent mit Gewebeentnahmen belastet werden können, ist die derzeitige Verfügbarkeit von Zellmaterial, insbesondere humanen Ursprungs, unbefriedigend.

Primärzellen, also solche Zellen, die aus einem unmittelbar zuvor noch intakten Organ bzw. Gewebe isoliert wurden, haben den Nachteil, dass ihre Teilungsfähigkeit limitiert ist. In höheren Passagen tritt häufig die sogenannte Zellseneszenz auf. Dies gilt insbesondere für humane Primärzellen. In diesem Zusammenhang wird von der sogenannten Hayflick-Grenze gesprochen, mit der die bei Eukaryoten begrenzte Anzahl von Zellteilungen bezeichnet wird, denen sich eine Zelle unterziehen kann. Bei Erreichen dieser Grenze wird der programmierte Zelltod eingeleitet, weil die Telomere eine kritische reduzierte Länge erreicht haben.

Einen gewissen Vorteil bieten Stammzellen, die anfangs eine starke Proliferationsfähigkeit aufweisen, deren Verfügbarkeit jedoch auch durch das Auftreten der Zellseneszenz in höheren Passagen beschränkt wird. Außerdem verlieren Stammzellen, insbesondere solche mesenchymalen Charakters, in hohen Passagen immer stärker ihre Differenzierungsfähigkeit und sind deshalb nur beschränkt für die eingangs genannten Zwecke und insbesondere für Untersuchungen der Differenzierungsprozesse verwendbar.

Um diese Limitierungen zu überwinden, wurden immortalisierte Zelllinien aus verschiedenen Gewebetypen generiert. Unter "Immortalisierung" wird das Unsterblich-Machen von Zellen verstanden. Die Begrenzung der Anzahl von Zellteilungen durch die Hayflick-Grenze wird dadurch aufgehoben. Immortalisierte Zellen bzw. Zelllinien können sich im Gegensatz zu gewöhnlichen Zellen beliebig häufig teilen und lassen sich in Zellkultur unbegrenzt vermehren. Eine Immortalisierung kann beispielsweise durch eine Infektion der Zellen mit bestimmten Viren, d.h. durch virale Transduktion, oder durch Fusion mit Tumorzellen erreicht werden. Häufig behalten immortalisierte Zellen die Zellfunktionen der primären Zellen, aus denen sie generiert wurden, bei.

Derzeit stehen nur relativ wenige immortalisierte Zellen zur Verfügung, meist murine, porcine und aus dem Knochenmark entnommene und anschließend immortalisierte humane mesenchymale Stammzellen (BMMSCs). Weiterhin wurden Stammzellen aus fetalem porcinem Pankreas, murinen embryonalen Fibroblasten und aus murinem Fettgewebe isoliert und anschließend immortalisiert; vgl. Cao et al. (2011), "Characterization of immortalized mesenchymal stem cells derived from foetal procine pancreas" Cell Prolif 44(1):19-32; Gong et al. (2011), "Immortalized mesenchymal stem cells: an alternative to primary mesenchymal stem cells in neuronal differentiation and neuroregeneration associated studies" Journal of Biomedical Science 18; Yalvac et al. (2011), "Differentiation and neuro-protective properties of immortalized human tooth germ stem cells" Neurochem Res 36(12):2227-2235; Huang et al. (2012), "Conditionally Immortalized Mouse Embryonic Fibroblasts Retain Proliferative Activity without Comprising Multipotent Differentiation Potential" Plos One 7(2); Komine et al. (2012), "Establishment of adipose-derived mesenchymal stem cell lines from a p53-knockout mouse" Biochem Biophys Res Commun 426(4):468-474; Moscoso et al. (2012), "Immortalization of bone marrow-derived porcine mesenchymal stem cells and their differentiation into cells expressing cardiac phenotypic markers" Journal of Tissue Engineering and Regenerative Medicine 6(8):655-665; Sreejit et al. (2012), "Generation of mesenchymal stem cell lines from murine bone marrow" Cell Tissue Res 350(1):55-68; Ramakrishnan et al. (2013), "Primary marrow-derived stromal cells: isolation and manipulation" Methods Mol Biol 1035: 75-101; Zamperone et al. (2013), "Isolation and Characterization of a Spontaneously Immortalized Multipotent Mesenchymal Cell Line Derived from Mouse Subcutaneous Adipose Tissue" Stern Cells Dev.

In der WO 02/34891 und in Darimont et al. (2002), Cell Growth and Differentiation, Bd. 13, Nr. 2, S. 59-67 werden eine als hPOBtert bezeichnete humane immortalisierte Zellinie offenbart, die nach Angaben der Autoren ostogen differenzierbar sei. Sie leitet sich aus dem Periosteum einer 13jährigen Patientin ab. Die WO 99/39724 beschreibt eine immortalisierte Periostzelllinie aus dem Femur.

Es besteht demnach weiterhin ein großer Bedarf an immortalisierten Zellen.

Vor diesem Hintergrund ist es eine Aufgabe der vorliegenden Erfindung eine weitere immortalisierte Zelle bzw. eine eine solche Zelle aufweisende immortalisierte Zelllinie und Zellkultur bereitzustellen, um den Bedarf im Stand der Technik zu befriedigen.

Diese Aufgabe wird durch die Bereitstellung einer immortalisierten Schädelperiostzelle, einer immortalisierten Schädelperiostzelllinie und einer Zellkultur, die immortalisierte Schädeleriostzellen aufweist, gelöst.

Als "Periost" oder auch "Knochenhaut" wird die den Knochen bedeckende, bindegewebsartige Hülle bezeichnet. Das Periost dient dem Schutz des Knochens, außerdem ist es die Anheftungsstelle für Bänder und Sehnen. Das Periost besteht aus einer äußeren Kollagenschicht mit elastischen Fasern sowie einer inneren Schicht, die Nerven und Blutgefäße sowie mesenchymale Vorläuferzellen enthält. Die Vorläuferzellen können nach entsprechender Stimulation in verschiedene Richtungen differenzieren. Dem Periost kommt eine wichtige Bedeutung in der natürlichen Knochenheilung zu.

Die der Erfindung zugrundeliegenden Aufgaben werden hiermit vollkommen gelöst.

Die Erfinderin stellt erstmals eine immortalisierte Schädelperiostzelle bzw. eine aus immortalisierten Schädelperiostzellen gebildete immortalisierte Periostzelllinie bzw. -zellkultur bereit. Die erfindungsgemäßen Zellen erlauben die Durchführung von Experimenten und anwendungsbezogenen Einsätzen, ohne dass patientenbezogene Schwankungen auftreten, die aus der Verwendung von Primärzellen bekannt sind. Die Reproduzierbarkeit der Ergebnisse wird dadurch erhöht. Die erfindungsgemäßen Zellen sind einfacher als Primärzellen in der Handhabung. Ferner tritt das Problem der Zellseneszenz nicht auf. Die erfindungsgemäßen Zellen proliferieren unbegrenzt.

Die erfindungsgemäße immortalisierte Schädelperiostzelle eignet sich insbesondere zur Untersuchung der Osteogenese und Mineralisierung, aber auch zur Erforschung von Prozessen der Zelladhäsion, Proliferation und Apoptose sowie der Wundheilung.

Erfindungsgemäß wird unter einer "immortalisierten Periostzelle" eine solche Zelle verstanden, die durch die Immortalisierung von Periostzellen bzw. von aus dem Periost stammenden Zellen erhalten wurde. "Immortalisierung" bezeichnet das Unsterblich-Machen von Zellen, bspw. durch virale Infektion, Transduktion mit Oncogenprodukten wie dem großen T-Antigen von SV40 oder durch Fusion mit Tumorzellen, wie weiter oben beschrieben.

Bei der immortalisierten Periostzelle kann es sich um eine humane immortalisierte Schädelperiostzelle handeln.

Diese Maßnahme hat den Vorteil, dass die erfindungsgemäße Zelle besonders gut für Untersuchungen geeignet ist, die Rückschlüsse auf Vorgänge im menschlichen Organismus erlauben sollen. Dabei bedeutet in diesem Zusammenhang "human", dass die primären Periostzellen, die immortalisiert werden, humanen Ursprungs sind.

Bei der immortalisierten Periostzelle handelt es sich um eine immortalisierte Schädelperiostzelle.

Hier werden die Primärzellen aus dem Periost des Schädelknochens immortalisiert. Diese Maßnahme hat den Vorteil, dass eine nach Erkenntnissen der Erfinderin besonders geeignete immortalisierte Periostzelle bereitgestellt wird.

Ferner ist es erfindungsgemäß bevorzugt, wenn die immortalisierte Periostzelle osteogen differenzierbar ist.

Erfindungsgemäß bedeutet "osteogen differenzierbar", dass die Zelle das Potenzial aufweist, sich in eine knochengewebebildende Zelle zu entwickeln. Osteogen differenzierte Zellen sind u.a. charakterisiert durch die Genexpression von Osteogenesemarkern wie die Transkriptionsfaktoren Osterix und Runx-2 oder des Peptidhormons Osteocalcin. Die osteogene Differenzierung kann bspw. durch die Inkubation der erfindungsgemäßen Zelle mit knochenspezifischen Wachstumsfaktoren und Zytokinen induziert werden. Diese Maßnahme hat den Vorteil, dass die erfindungsgemäße Zelle bzw. Zellkultur besonders geeignet ist, in der Grundlagenforschung Prozesse der Osteogenese, Mineralisation und Knochenheilung zu analysieren und entschlüsseln.

Ein weiterer Gegenstand der vorliegenden Erfindung betrifft eine immortalisierte Schädelperiostzelle bzw. immortalisierte Schädelperiostzelllinie bzw. -zellkultur, die am 29. November 2013 bei der Deutschen Sammlung von Mikroorganismen und Zellkulturen GmbH, Inhoffenstraße 7B, 38124 Braunschweig, Deutschland (DSMZ) unter der Hinterlegungsnummer DSM ACC3218 und der Bezeichnung "Tag58" hinterlegt wurde.

Diese Maßnahme hat den Vorteil, dass eine solche erfindungsgemäße Schädelperiostzelle bzw. Schädelperiostzelllinie bereitgestellt wird, die besondere und überraschende Eigenschaften aufweist. Die hinterlegte erfindungsgemäße Zelllinie wurde aus humanen primären Schädelperiostzellen generiert. Diese wurden lentiviral mit dem großen T-Antigen des SV40-Virus transduziert. Gemäß dem verwendeten Transduktionsprotokoll wurde eine konditionierte Immortalisierung der Zellen erwartet. Demnach sollten die immortalisierten Kieferperiostzellen bei 33°C im immortalisierten Zustand verbleiben, während bei höheren Temperaturen von beispielsweise 37°C bis 39°C eine Inaktivierung des großen T-Antigens erwartet wurde und die Zellen wieder ihren "Primärzustand" erreichen sollten. Überraschenderweise stellte sich jedoch heraus, dass die hinterlegten Zellen dauerhaft und unabhängig von der Kultivierungstemperatur immortalisiert sind. Ferner zeigte sich überraschenderweise, dass die hinterlegten Zellen noch stärker osteogen differenzierbar sind als die primären Schädelperiostzellen, aus denen sie gewonnen wurden. Die hinterlegten erfindungsgemäßen Zellen sind deshalb für die beabsichtigten Zwecke besonders geeignet.

Vor diesem Hintergrund betrifft die Erfindung auch die Verwendung einer immortalisierten Schädelperiostzelle *in vitro* zur Untersuchung der Osteogenese, Mineralisation und Knochenheilung, wobei es sich vorzugsweise um die Verwendung der erfindungsgemäßen immortalisierten Periostzelle handelt.

Die Eigenschaften, Merkmale und Vorteile der erfindungsgemäßen immortalisierten Schädelperiostzelle gelten für die erfindungsgemäße immortalisierte Schädelperiostzelllinie und die erfindungsgemäße Zellkultur sowie die erfindungsgemäße Verwendung gleichermaßen.

Ein weiterer Gegenstand der vorliegenden Erfindung betrifft ein Verfahren zur Herstellung einer immortalisierten Schädelperiostzelle, das folgende Schritte aufweist:
1. Bereitstellen einer isolierten Schädelperiostprimärzelle, und
2. Immortalisierung der Periostzelle zum Erhalt einer immortalisierten Schädelperiostzelle.

Mit dem erfindungsgemäßen Verfahren lässt sich reproduzierbar eine erfindungsgemäße immortalisierte Schädelperiostzelle bzw. -zelllinie herstellen. Die Immortalisierung kann dabei mittels dem Fachmann bekannter Methoden erfolgen, beispielsweise durch das Einbringen von Onkogenen oder das Ausschalten von Tumorsuppressorgenen. Immortalisierung kann ferner durch Infektion der Schädeleriostprimärzelle mit bestimmten Viren, wie beispielsweise dem SV40-Virus, erfolgen.

Dabei ist es bevorzugt, wenn die Immortalisierung durch Transduktion des großen T-Antigens des SV40-Virus in die Schädelperiostprimärzelle erfolgt.

Diese Maßnahme hat den Vorteil, dass ein etabliertes Verfahren zur Generierung einer immortalisierten Schädelperiostzelle bzw. -zelllinie zum Einsatz kommt, das sich nach Erkenntnissen der Erfinderin besonders eignet.

Ein weiterer Gegenstand der vorliegenden Erfindung betrifft eine immortalisierte Schädelperiostzelle, die erhältlich ist über das erfindungsgemäße Verfahren zur Herstellung einer immortalisierten Schädelperiostzelle.

Die Merkmale, Eigenschaften und Vorteile der eingangs erwähnten immortalisierten Schädelperiostzelle gelten für die derart erhältliche immortalisierte Schädelperiostzelle gleichermaßen.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

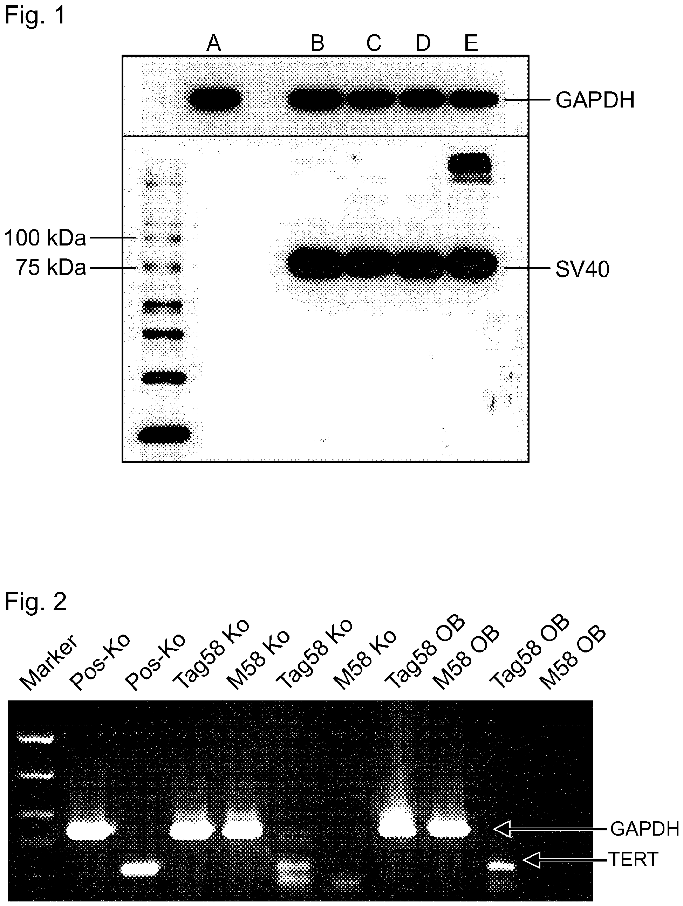

Die vorliegende Erfindung wird nun anhand von Ausführungsbeispielen näher erläutert, aus denen sich weitere Eigenschaften, Merkmale und Vorteile ergeben. Die Ausführungsbeispiele sind rein illustrativ und schränken die Reichweite der Erfindung nicht ein. Dabei wird Bezug genommen auf die beigefügten Figuren, in denen Folgendes dargestellt ist
- Fig. 1: zeigt einen Westernblot zum Nachweis des permanenten Immortalisierungszustandes der erfindungsgemäßen Periostzellen ("Tag58") über die Detektion des großen T-Antigens von SV40. A: Primäre Schädelperiostzellen ("M58") bei 37°C; B: TAg58 bei 33°C; C: TAg58 bei 37°C; D: TAg58 bei 38°C; E: TAg58 bei 39°C.
- Fig. 2: zeigt das Ergebnis einer quantitativen PCR zum Nachweis der Genexpression der humanen Telomerase Reverse Transcriptase (hTERT). Pos-Ko: Positivkontrolle; Tag58 Ko: Tag58 undifferenziert; M58 Ko: M58 undifferenziert; TAg58 OB: Tag58 osteogen stimuliert (Tag 10); M58 OB: M58 osteogen stimuliert (Tag 10).

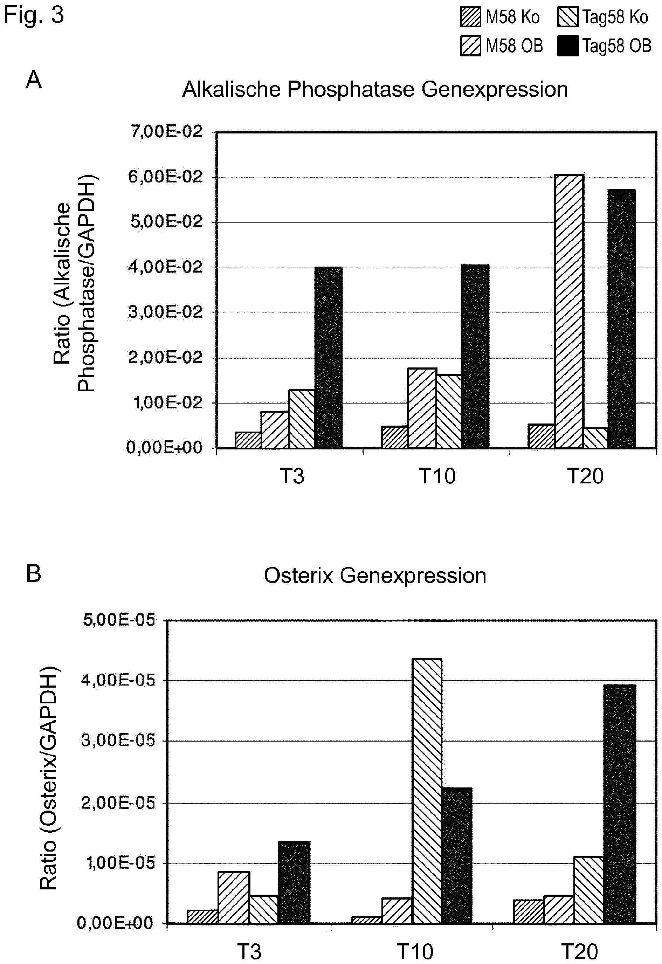

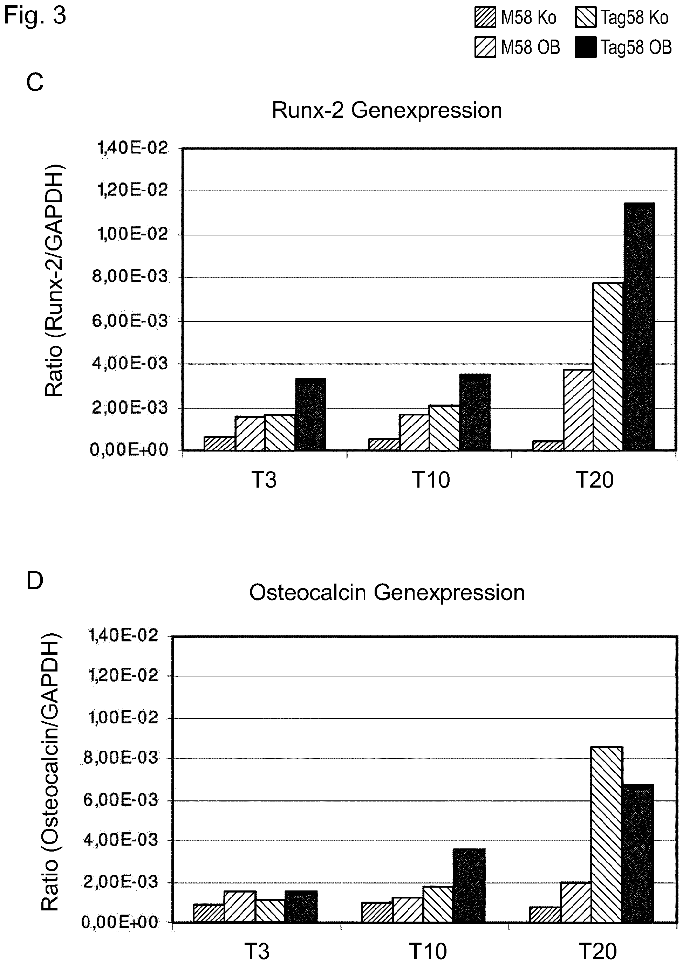

- Fig. 3: zeigt das Ergebnis einer quantitativen PCR zum Nachweis der Genexpression von Osteogenese-relevanten Markern. A: Alkalische Phosphatase; B: Osterix; C: Runx-2; D: Osteocalcin. Abgebildet ist das Verhältnis der Expressionsstärke des jeweiligen Gens zu der des Haushaltsgens GAPDH am Tag 3, 10 und 20 der Osteogenese.

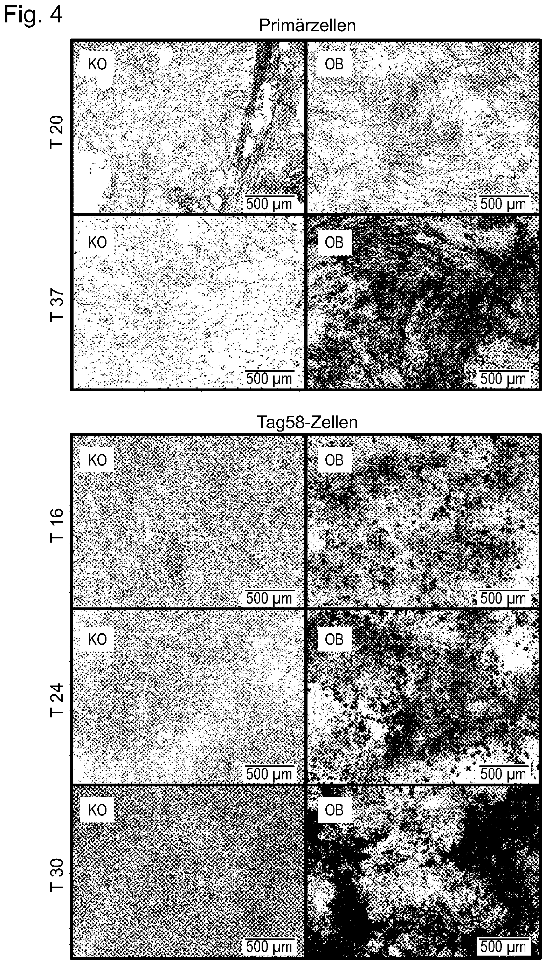

- Fig. 4: zeigt das Ergebnis einer mikroskopischen Untersuchung zum Nachweis von Kalziumphosphatpräzipitaten mit Hilfe der Alizarin-Färbung zu verschiedenen Untersuchungszeitpunkten der Osteogenese (Tag 16, T 16; Tag 20, T 20; Tag 24, T 24; Tag 30, T 30; Tag 37, T 37). KO: Undifferenzierte Zellen; OB: Osteogen stimulierte Zellen.

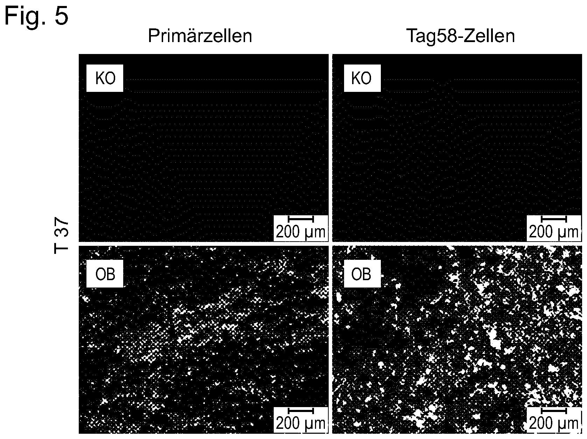

- Fig. 5: zeigt das Ergebnis einer mikroskopischen Untersuchung mit Hilfe der Osteolmage-Färbung am Tag 37 (T 37) der osteogenen Stimulation zum Nachweis der Kalziumphosphatpräzipitate. KO: Undifferenzierte Zellen; OB: Osteogen stimulierte Zellen.

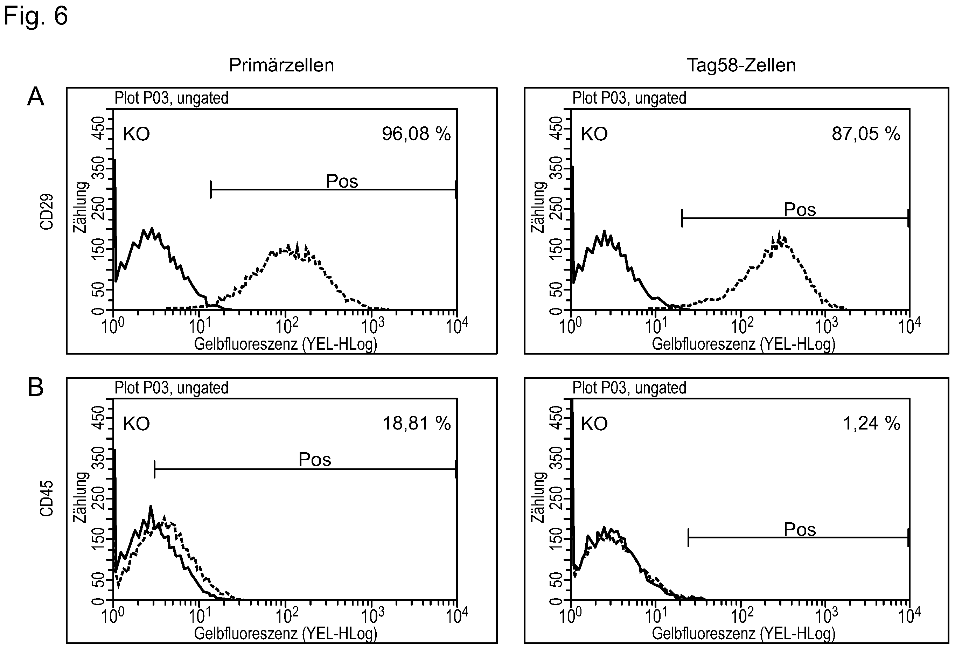

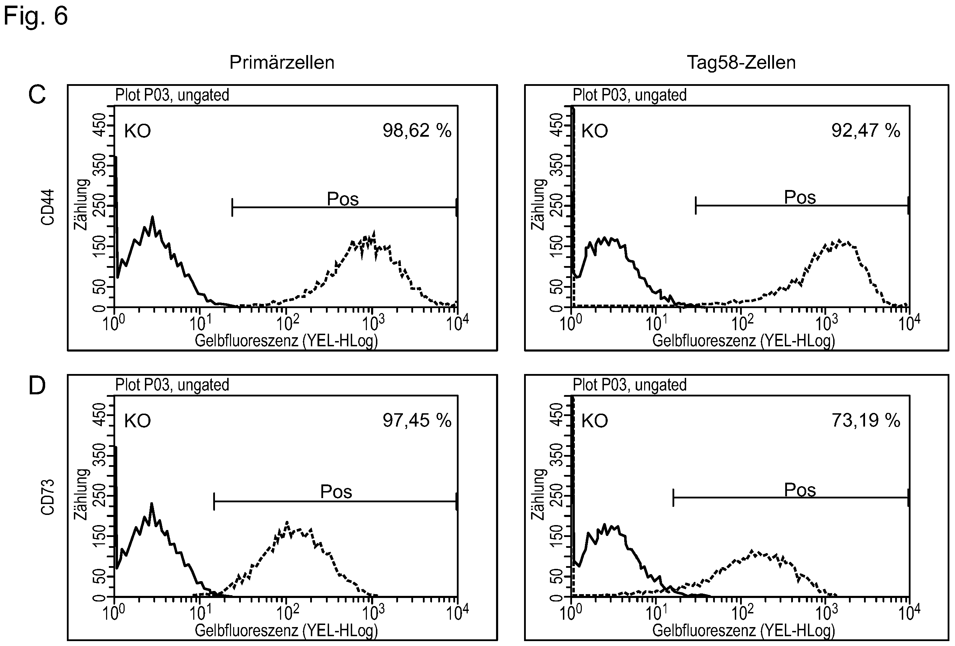

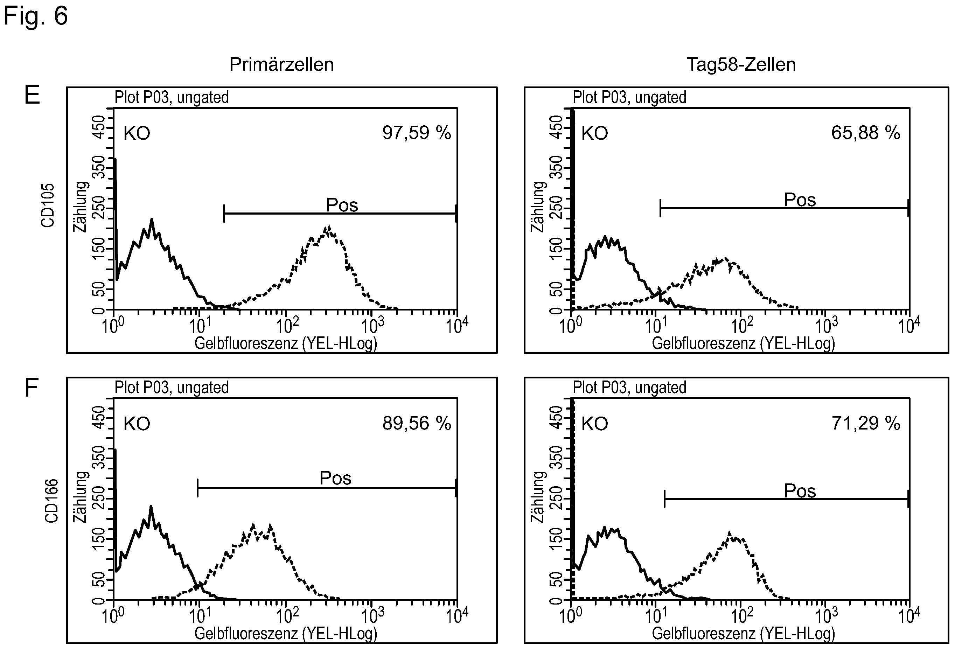

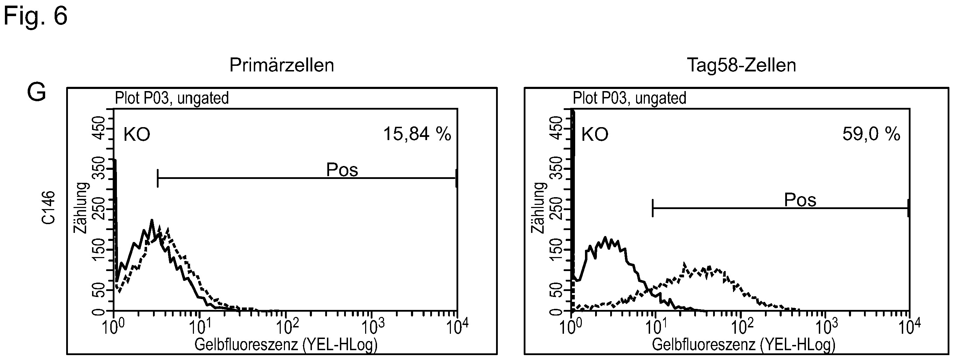

- Fig. 6: zeigt das Ergebnis einer durchflusszytometrischen Untersuchung zum Nachweis charakteristischer Oberflächenantigene der Zelllinie im Vergleich zu den Parentalzellen.

### Ausführungsbeispiele

### 1. Material und Methoden

### Lentivirale Transduktion von primären Schädelperiostzellen

Primäre Schädelknochenhautzellen bzw. Schädelperiostzellen (8 Aliquote a 5,0 x 10⁶ Zellen) wurden auf Trockeneis an die Firma Sirion Biotech, Martinsried, Deutschland, verschickt und von dieser lentiviral transduziert. Dies erfolgte unter Verwendung des Vektors Lenti_pCDH-CMV-LTtsA58-EF1-Neo - replic.-defic., self-inactivating 3rd generation lentiviral vector.

72 Stunden nach der Transduktion wurde die Selektion der transduzierten Zellen mit 0,50 mg/ml G418 begonnen. Unter diesem Selektionsdruck wurden die Zellen für 10 Tage kultiviert und anschließend kryokonserviert (im Cryo-SFM Medium von Promocell, C-29912). Nicht-transduzierte Zellen starben nach 5 Tagen der Kultivierung im Geneticin-(G418)-enthaltenen Medium ab. Nach dem Auftauen, behalten die Zellen bei 37°C den Immortalisierungszustand bei.

Die Induktion zur osteogenen Differenzierung erfolgt durch die Zugabe von 4 µM Dexamethason, 10 mM ß-Glycerophosphate Disodium Salt Hydrate und 100 µM Ascorbinsäure (L-ascorbic acid 2-phosphate). Die Bildung der Kalziumphosphatpräzipitate durch das Zellmonolayer wird bereits nach 15 Tagen sichtbar.

Wie bereits oben beschrieben, wurden die Primärzellen aus humaner Schädelknochenhaut isoliert. Das Gewebe wurde erst mechanisch zerkleinert, danach erfolgte der enzymatische Verdau des Gewebes mit Typ XI Kollagen für 90 min. Die vereinzelten Zellen wurden in 75 cm² Gewebekulturflaschen in DMEM:Ham's F12 (1:1 mixture) + 10% FCS + 1% PenStrep + Fungizide kultiviert.

Das exprimierte Transgen ist die cDNA des großen T-Antigens (LTtsA58) des Polyomavirus SV40. 72 Stunden nach der Transduktion der primären Schädelperiostzellen wurden diese für 10 Tage in Anwesenheit der antibakteriell wirksamen Substanz Geneticin (G-418; 0,5 mg/ml) selektioniert und anschließend kryokonserviert.

Die Zellen wurden einem Immortalisierungsprotokoll unterzogen, das zu einer konditionierten Immortalisierung führen sollte. Demnach wurde erwartet, dass eine Zellkultivierung bei 33°C für die Beibehaltung des Immortalisierungszustandes notwendig sei. Bei höheren Temperaturen von ca. 37 bis 39°C sollten das Transgen deaktiviert werden und die Zellen ihren "Primärzustand" wieder erlangen. Dies ließ sich jedoch überraschenderweise in den Versuchen der Erfinderin nicht nachweisen. Die Zellen zeigten einen Immortalisierungszustand unabhängig von der Kultivierungstemperatur.

Zur der besseren Vergleichbarkeit der Ergebnisse wurden sowohl die immortalisierten als auch die Primärzellen bei 37°C kultiviert und untersucht.

So generierte immortalisierte Schädelperiostzellen wurden eingefroren und an das Leibnitz-Institut - Deutsche Sammlung von Mikroorganismen und Zellkulturen GmbH (DSMZ), Inhoffenstraße 7B, 38124 Braunschweig, Deutschland geschickt. Dort wurde die Lebensfähigkeit und Reinheit des Materials bestätigt. Die Zellkultur mit der Bezeichnung "Tag58" wurde bei der DSMZ offiziell am 29. November 2013 unter der Hinterlegungsnummer DSM ACC3218 hinterlegt.

### 2. Ergebnisse

### Nachweis des Immortalisierungszustandes mittels Westernblots

Um nachweisen zu können, dass die generierten Tag58-Zellen tatsächlich immortalisiert sind, wurde das große T-Antigen von SV40 auf Proteinebene mittels Westernblots in den Lysaten der Zellen detektiert.

Aufgrund der anvisierten konditionierten Immortalisierung wurde davon ausgegangen, dass das große T-Antigen von SV40 nur in bei 33°C kultivierten Zellen und nicht bei anderen Inkubationstemperaturen, wie beispielsweise 37°C, 38°C oder 39°C nachzuweisen ist. Dies ließ sich jedoch nicht bestätigen (siehe Fig. 1). Das SV40 T-Antigen war in allen Proben der immortalisierten Zelllinie unabhängig von der Inkubationstemperatur, nachweisbar (Spuren B bis E). In dem Zelllysat der parentalen Primärzellen war kein spezifisches SV40 T-Antigensignal detektierbar (Spur A).

Die erwartete Größe der spezifischen SV40 T-Antigenbande von ca. 80 kDa wurde bestätigt. Als interne Kontrolle wurde das Haushaltsprotein GAPDH verwendet.

### Nachweis der Genexpression der humanen Telomerase reverse Transkriptase (hTERT) mittels quantitativer PCR

Um zu testen, ob das ins Genom integrierte SV40 große T-Antigen tatsächlich funktionell aktiv ist, wurde die Genexpression von p53 und hTERT mittels quantitativer PCR untersucht. Dazu wurde die erfindungsgemäßen Tag58-Zellen osteogen stimuliert. Die Induktion zur osteogenen Differenzierung erfolgte durch die Zugabe von 4 µM Dexamethason, 10 mM ß-Glycerophosphate Disodium Salt Hydrate und 100 µM Ascorbinsäure (L-ascorbic acid 2-phosphate). In den mRNA-Spiegeln von p53 ließen sich keine nennenswerten Unterschiede in den Tag58-Zellen im Vergleich zu den parentalen M58-Primärzellen detektieren. Hierzu im Gegensatz ließ sich die hTERT-Genexpression jedoch nur in den Tag58-Zellen nachweisen (siehe Fig. 2). Die spezifische hTERT-Bande zeigte in den osteogen stimulierten Tag58-Zellen am Tag 10 (Tag58 OB) ein noch stärkeres Signal.

Sowohl in den undifferenzierten (M58 Ko) als auch den osteogen stimulierten parentalen M58-Zellen (M58 OB) wurden kein spezifisches Signal für hTERT detektiert.

### Nachweis der Genexpression von Osteogenese-relevanten Markern mittels quantitativer PCR

Die Genexpression des frühen Osteogenese Markers Alkalische Phosphatase sowie der Transkriptionsfaktoren Osterix und Runx-2 und des späten Osteogenese Markers Osteocalcin wurde mittels PCR quantifiziert. Untersucht wurde die Expression zu Beginn (Tag 3; T3), in der Mitte (Tag 10; T10) und am Ende (Tag 20; T20) der osteogenen Differenzierung.

Wie der Fig. 3A zu entnehmen ist, waren am Anfang und in der Mitte der Osteogenese (Tag 3 und 10) sowohl höhere Grundspiegel (Ko) als auch höhere Induktionen (OB) der alkalischen Phosphatase in den Tag58-Zellen durch die PCR nachweisbar. Am Ende der Differenzierung (Tag 20) glichen sich die mRNA-Spiegel wieder an.

Zu allen untersuchten Zeitpunkten der Osteogenese konnten höhere Grundspiegel (Ko) des Transkriptionsfaktors Osterix in den immortalisierten Zellen im Vergleich zu den Primärzellen detektiert werden (Fig. 3B). Mit Ausnahme von Tag 10 wurden auch höhere Induktionen (OB) in den Tag58-Zellen nachgewiesen.

Die quantitative Untersuchung der Runx-2-Genexpression zeigte zu allen drei untersuchten Zeitpunkten der Osteogenese höhere Spiegel in den Tag58-Zellen im Vergleich zu den parentalen Primärzellen M58 (Fig. 3C).

Die Analyse der Osteocalcin-Genexpression ergab nur an Tag 10 und 20 höhere Grundspiegel in den Tag58-Zellen, wie der Fig. 3D zu entnehmen ist.

### Colorimetrischer Nachweis der Proliferationsaktivität

Der Nachweis der Proliferationsaktivität erfolgte mit Hilfe eines semiquantitativen MTT-basierten colorimetrischen Assays (EZ4U, Biozol). Durch die Umsetzung der Tetrazoliumsalze zu Formazanderivaten in den Mitochondrien der Zelle können Rückschlüsse auf die Zellvitalität gezogen werden. Die Ergebnisse der gemessenen optischen Dichten sind in der nachfolgenden Tabelle 1 zusammengefasst.

**Tab 1 Proliferationsaktivitäten der Primär- (M58) versus Tag58 Zellen im undifferenzierten bzw. differenzierten Stadium, ermittelt mithilfe eines colorimetrischen Assays (EZ4U, Biozol). Aufgelistet sind die optischen Dichten ± Standardabweichung (n=3).**

| | Primärzellen (M58) | | Tag58-Zellen (Tag58) | |
|---|---|---|---|---|
| | *undifferenziert* | *osteogen stimuliert* | *undifferenziert* | *osteogen stimuliert* |
| Tag 3 | 0,419 ± 0,096 | 0,428 ± 0,051 | 0,569 ± 0,092 | 0,595 ± 0,232 |
| Tag 10 | 1,527 ± 0,457 | 0,823 ± 0,100 | 2,222 ± 0,166 | 2,023 ± 0,185 |
| Tag 20 | 2,390 ± 0,181 | 1,621 ± 0,247 | 2,090 ± 0,326 | 1,889 ± 0,145 |

Dabei ließ sich Folgendes feststellen: Am Tag 3 der osteogenen Differenzierung gab es keine signifikanten Unterschiede zwischen den parentalen Primärzellen (M58) und den Tag58-Zellen. Die Werte der optischen Dichte waren tendenziell höher in den Tag58-Zellen, erreichten jedoch kein Signifikanzniveau verglichen mit den parentalen Primärzellen.

Am Tag 10 der Osteogenese zeigten sich jedoch signifikante Unterschiede der Proliferationsaktivität. Während osteogen stimulierte Primärzellen (M58) ihre Proliferationsaktivität auf halbhohe OD-Werte reduzieren, sind die Proliferationsraten der undifferenzierten und differenzierten Tag58-Zellen nicht signifikant unterschiedlich, liegen jedoch weit über den aus Primärzellen ermittelten OD-Werten.

Am Tag 20 der Osteogenese konnten keine nennenswerten Unterschiede zwischen den Primär- und Tag58-Zellen detektiert werden.

### Nachweis des Mineralisationspotentials

Mithilfe der Alizarin- oder Fluoreszenz-basierten Osteolmage-Färbung können Kalziumphosphatpräparate nachgewiesen werden (Fig. 3, Fig. 4), die von mesenchymalen Vorläuferzellen im Laufe des osteogenen Differenzierungsprozesses gebildet werden.

Mithilfe der Alizarin-Färbung (Fig. 4) konnte nachgewiesen werden, dass in den Tag58-Zellen die Bildung der Kalziumphosphatpräpizitate früher einsetzt als in den parentalen Primärzellen M58. Diese beginnt in den immortalisierten Tag58-Zellen bereits nach 10 Tagen der osteogenen Stimulation und ist nach 16 Tagen (T 16) mittels Alizarin-Färbung deutlich nachweisbar (siehe Fig. 4). Im Gegensatz dazu war eine beginnende Mineralisierung in den Primärzellen M58 erst nach dem Tag 30 der osteogenen Stimulation sichtbar und konnte am Tag 37 (T 37) mittels Alizarin-Färbung detektiert werden.

In einem synchronisierten Experiment konnte am Tag 37 (T 37) eine intensivere Osteolmage-Färbung der Kalziumphosphatpräzipitate in dem Monolayer der Tag58-Zellen nachgewiesen werden, verglichen mit derjenigen der Primärzellen M58 (Fig. 5).

### Durchflusszytometrische Untersuchungen der Oberflächenantigenexpression

Verschiedene Oberflächenantigene wie CD29, CD44, CD73, CD90, CD105 und CD166 wurden bereits als Stammzellmarker der mesenchymalen Stammzellreihe definiert. Untersucht wurde die Oberflächenmarkerexpression 10 Tage nach der Aussaat der Zellen. CD45 ist ein Leukozytenantigen, das in Schädelperiostzellen meistens negativ ist. Die Parentalzellen M58, aus denen die erfindungsgemäßen Tag58-Zellen stammen, zeigen jedoch eine schwache CD45-Expression (18,81%, siehe Fig. 5B). Die Tag58-Zellen sind jedoch nahezu negativ bezüglich der CD45-Expression. Die CD44-, CD73-, CD105-, CD166-Expressionen zeigten sich in den Primär- sowie in den immortalisierten Zellen relativ vergleichbar (Fig. 5C-F), mit der Einschränkung, dass der prozentuale Anteil der positiven Tag58 für CD73, CD105 und CD166 etwas niedriger war. Überraschenderweise wurde eine dreifach höhere CD146-Expression in den TAg58-Zellen im Vergleich zu den Primärzellen detektiert (siehe Fig. 5G).

### 3. Fazit

Die Erfinderin konnte mittels einer Vielzahl von experimentellen Nachweisen bestätigen, dass ausgehend von humanen primären Schädelperiostzellen eine immortalisierte Periostzelllinie generiert wurde.

## Patentansprüche

1. Immortalisierte Schädelperiostzelle.

2. Immortalisierte Schädelperiostzelle nach Anspruch 1, die osteogen differenzierbar ist.

3. Immortalisierte Schädelperiostzelle nach Anspruch 1 oder 2, die am 29. November 2013 bei der Deutschen Sammlung von Mikroorganismen und Zellkulturen GmbH, Braunschweig, Deutschland (DSMZ) unter der Hinterlegungsnummer DSM ACC3218 und der Bezeichnung "Tag58" hinterlegt wurde.

4. Immortalisierte Schädelperiostzellinie, die die Schädelperiostzelle nach einem der Ansprüche 1 bis 3 aufweist.

5. Zellkultur, die die Schädelperiostzelle nach einem der Ansprüche 1 bis 4 und/oder die Schädelperiostzellinie nach Anspruch 4 aufweist.

6. In vitro Verfahren zur Herstellung einer immortalisierten Schädelperiostzelle, das folgende Schritte aufweist:
1. Bereitstellen einer isolierten Schädelperiostprimärzelle, und
2. Immortalisierung der Schädelperiostprimärzellezum Erhalt einer immortalisierten Schädelperiostzelle.

7. Verfahren nach Anspruch 6, **dadurch gekennzeichnet, dass** die Immortalisierung durch Transduktion des großen T-Antigens des SV40-Virus in die Schädelperiostprimärzelleerfolgt.

8. Verwendung einer immortalisierten Schädelperiostzelle *in vitro* zur Untersuchung der Osteogenese, Mineralisation und Knochenheilung.

9. Verwendung nach Anspruch 8, **dadurch gekennzeichnet, dass** als immortalisierte Schädelperiostzelle die immortalisierte Schädelperiostzelle nach einem der Ansprüche 1 bis 3 verwendet wird.

## Claims

1. An immortalized cranium periosteum cell.

2. The immortalized cranium periosteum cell of claim 1, which is osteogenetically differentiable.

3. The immortalized cranium periosteum cell of claim 1 or 2, which was deposited on 29 November 2013 under the designation "Tag58" at the Deutsche Sammlung von Mikroorganismen und Zellkulturen GmbH, Braunschweig, Deutschland (DSMZ), under the accession number DSM ACC3218.

4. An immortalized cranium periosteum cell line, which comprises the cranium periosteum cell of any of the claims 1 to 3.

5. A cell culture, which comprises the cranium periosteum cell of any of claims 1 to 4 and/or the immortalized cranium periosteum cell line of claim 4.

6. An *in vitro* method for the production of an immortalized cranium periosteum cell, comprising the following steps:
1. Providing an isolated cranium periosteum primary cell, and
2. Immortalizing the cranium periosteum primary cell for obtaining an immortalized cranium periosteum cell.

7. The method of claim 6, **characterized in that** the immortalization occurs by transduction of the large T antigen of the SV40 virus into the cranium periosteum primary cell.

8. A use of an immortalized cranium periosteum cell *in vitro* for the examination of the osteogenesis, mineralization and bone healing.

9. The use of claim 8, **characterized in that** the immortalized cranium periosteum cell is the immortalized cranium periosteum cell of any of claims 1 to 3.

## Revendications

1. Cellule immortalisée de périoste crânien.

2. Cellule immortalisée de périoste crânien selon la revendication 1, qui peut être différenciée d'un point de vue ostéogénique.

3. Cellule immortalisée de périoste crânien selon la revendication 1 ou 2, qui a été déposée le 29 novembre 2013 auprès de la Deutsche Sammlung von Mikroorgnismen und Zellkulturen GmbH, Braunschweig, Allemagne (DSMZ) sous le numéro de dépôt DSM ACC3218 et la désignation « Tag58 ».

4. Lignée cellulaire immortalisée de périoste crânien qui présente la cellule de périoste crânien selon l'une quelconque des revendications 1 à 3.

5. Culture cellulaire qui présente la cellule de périoste crânien selon l'une quelconque des revendications 1 à 4 et/ou la lignée cellulaire de périoste crânien selon la revendication 4.

6. Procédé *in vitro* de production d'une cellule immortalisée de périoste crânien qui présente les étapes suivantes :
1. fourniture d'une cellule primaire de périoste crânien isolée, et
2. immortalisation de la cellule primaire de périoste crânien pour obtenir une cellule immortalisée de périoste crânien.

7. Procédé selon la revendication 6, **caractérisé en ce que** l'immortalisation s'effectue par transduction de l'antigène grand-T du virus SV40 dans la cellule primaire de périoste crânien.

8. Utilisation d'une cellule immortalisée de périoste crânien *in vitro* pour étudier l'ostéogenèse, la minéralisation et la cicatrisation osseuse.

9. Utilisation selon la revendication 8, **caractérisée en ce qu'**est utilisée, en tant que cellule immortalisée de périoste crânien, la cellule immortalisée de périoste crânien selon l'une quelconque des revendications 1 à 3.
